# EUROPEAN PATENT APPLICATION

(11) **EP 2 871 174 A1**
(43) Date of publication of application: **13.05.2015**
(21) Application number: 13813620.5
(22) Date of filing: 28.06.2013
(51) Int. Cl.: C07C 63/28, B01D 53/02, B01J 20/22, B01J 20/30, C01B 31/20, C07D 213/16, C07D 213/22, C07F 1/08, F02M 21/00, F17C 11/00

(54) **METAL COMPLEX, AND ABSORBENT, OCCLUSION MATERIAL AND SEPARATION MATERIAL PRODUCED THEREFROM**

(30) Priority: 04.07.2012 JP 2012150840
(71) Applicant: Kuraray Co., Ltd., Okayama 710-0801 (JP)
(72) Inventor: INUBUSHI, Yasutaka, Kurashiki-shi Okayama 710-0801 (JP); IKEDA, Chikako, Kurashiki-shi Okayama 710-0801 (JP); HORI, Takashi, Kurashiki-shi Okayama 710-0801 (JP); YANE, Takako, Kurashiki-shi Okayama 710-0801 (JP)
(74) Representative: Müller-Boré & Partner Patentanwälte PartG mbB
(86) International application number: PCT/JP2013/067912
(87) International publication number: WO 2014/007181

(57) **Abstract**

An object is to provide a metal complex with high water resistance.

The above object is achieved by the metal complex comprising a multivalent carboxylic acid compound, a metal ion belonging to Groups 2 to 13 of the periodic table, an organic ligand capable of multidentate binding to the metal ion, and a monodentate organic ligand capable of binding to the metal ion. The metal complex has excellent gas adsorption, storage, and separation performance as well as excellent water resistance. The metal complex is stably present under high temperature and high humidity conditions, and can maintain high adsorption performance.

## Description

### Technical Field

### Cross Reference to Related Art

This application claims priority to Japanese Patent Application No. 2012-150840 filed on July 4, 2012, the disclosure of which is incorporated herein by reference in its entirety.

The present invention relates to a metal complex and to an adsorbent material, a storage material, and a separation material comprising the metal complex. More specifically, the present invention relates to a metal complex comprising a multivalent carboxylic acid compound, at least one metal ion, an organic ligand capable of multidentate binding to the metal ion, and a monodentate organic ligand capable of binding to the metal ion. The metal complex of the present invention is suitable for an adsorbent material, a storage material, or a separation material for adsorbing, storing, or separating carbon dioxide, hydrogen, carbon monoxide, oxygen, nitrogen, hydrocarbons having from 1 to 4 carbon atoms, noble gases, hydrogen sulfide, ammonia, organic vapor, or the like.

### Background Art

In the fields of deodorization, exhaust gas treatment, and the like, various adsorbent materials have so far been developed. Activated carbon is a representative example of these, and it has been used widely in various industries for the purpose of air cleaning, desulfurization, denitrification, or removal of harmful substances by making use of its excellent adsorption performance. In recent years, demand for nitrogen has been increasing, for example, in semiconductor manufacturing processes and the like. Such nitrogen is produced from air by using molecular sieving carbon according to the pressure swing adsorption process or temperature swing adsorption process. Molecular sieving carbon is also used for separation and purification of various gases such as purification of hydrogen from cracked methanol gas.

When a mixture of gases is separated according to the pressure swing adsorption process or temperature swing adsorption process, it is common practice to separate it based on the difference between the gases in equilibrium adsorption amount or rate of adsorption to molecular sieving carbon or zeolite used as a separation adsorbent material. When the mixture of gases is separated based on the difference in equilibrium adsorption amount, conventional adsorbent materials cannot selectively adsorb only the gas to be removed, and the separation coefficient decreases, making it inevitable that the size of the apparatus used increases. When the mixture of gases is separated into individual gases based on the difference in rate of adsorption, on the other hand, only the gas to be removed can be adsorbed, although it depends on the kind of gas. It is necessary, however, to alternately perform adsorption and desorption, and also in this case, the apparatus used should be larger.

On the other hand, a polymer metal complex has also been developed as an adsorbent material providing superior adsorption performance. The polymer metal complex has features including (1) large surface area and high porosity, (2) high designability, and (3) a change in dynamic structure when exposed to external stimulation. The polymer metal complex is expected to attain adsorption properties that known adsorbent materials do not have.

In practical application, however, in addition to further improvements in adsorption performance, storage performance, and separation performance, also desired has been improvement in resistance to water contained in actual gas (for example, see NPL 1).

### Citation List

### ▪ Non-patent Literature

NPL 1: J.J. Low, A.I. Benin, P. Jakubczak, J.F. Abrahamian, S.A. Faheem, R.R. Willis, Journal of the American Chemical Society, Volume 131, pp. 15834-15842 (2009)

### Summary of Invention

### Technical Problem

Accordingly, an object of the present invention is to provide a metal complex that can be used as a gas adsorbent material, a gas storage material, or a gas separation material having higher water resistance than conventional materials.

### Solution to Problem

As a result of intensive study, the present inventors found that the above object can be achieved by using a metal complex comprising a multivalent carboxylic acid compound, at least one metal ion, an organic ligand capable of multidentate binding to the metal ion, and a monodentate organic ligand capable of binding to the metal ion. The present invention was thus accomplished.

Specifically, the present invention provides the following.
(1) A metal complex comprising:
   a multivalent carboxylic acid compound,
   at least one metal ion selected from ions of metals belonging to Groups 2 to 13 of the periodic table,
   an organic ligand capable of multidentate binding to the metal ion, and
   a monodentate organic ligand capable of binding to the metal ion.
(2) The metal complex according to (1), wherein the monodentate organic ligand has a C₁₋₂₃ hydrocarbon group as a substituent.
(3) The metal complex according to (1) or (2), wherein the multivalent carboxylic acid compound is a dicarboxylic acid compound.
(4) The metal complex according to any one of (1) to (3), wherein the organic ligand capable of multidentate binding is at least one member selected from 1,4-diazabicyclo[2.2.2]octane, pyrazine, 2,5-dimethylpyrazine, 4,4'-bipyridyl, 2,2'-dimethyl-4,4'-bipyridine, 1,2-bis(4-pyridyl)ethyne, 1,4-bis(4-pyridyl) butadiyne, 1,4-bis(4-pyridyl)benzene, 3,6-di(4-pyridyl)-1,2,4,5-tetrazine, 2,2'-bi-1,6-naphthyridine, phenazine, diazapyrene, 2,6-di(4-pyridyl)-benzo[1,2-c:4,5-c']dipyrrole-1,3,5,7(2H,6H)-tetrone, N,N'-di(4-pyridyl)-1,4,5,8-naphthalene tetracarboxlic diimide, trans-1,2-bis(4-pyridyl)ethene, 4,4'-azopyridine, 1,2-bis(4-pyridyl)ethane, 4,4'-dipyridyl sulfide, 1,3-bis(4-pyridyl)propane, 1,2-bis(4-pyridyl)-glycol, and N-(4-pyridyl)isonicotinamide.
(5) The metal complex according to any one of (1) to (4), wherein the monodentate organic ligand is at least one member selected from 4-methylpyridine, 4-tert-butylpyridine, and 4-phenylpyridine.
(6) An adsorbent material comprising the metal complex according to any one of (1) to (5).
(7) The adsorbent material according to (6), wherein the adsorbent material is for adsorbing carbon dioxide, hydrogen, carbon monoxide, oxygen, nitrogen, hydrocarbons having from 1 to 4 carbon atoms, noble gases, hydrogen sulfide, ammonia, sulfur oxides, nitrogen oxides, siloxanes, or organic vapor.
(8) A storage material comprising the metal complex according to any one of (1) to (5).
(9) The storage material according to (8), wherein the storage material is for storing carbon dioxide, hydrogen, carbon monoxide, oxygen, nitrogen, hydrocarbons having from 1 to 4 carbon atoms, noble gases, hydrogen sulfide, ammonia, or organic vapor.
(10) A gas storage device comprising a pressure-resistant container that can be hermetically sealed and that has an inlet and outlet for gas, the pressure-resistant container having a gas storage space inside, and the storage material according to (8) being placed in the gas storage space.
(11) A gaseous-fuel vehicle comprising an internal combustion engine that obtains driving force from fuel gas supplied from the gas storage device according to (10).
(12) A separation material comprising the metal complex according to any one of (1) to (5).
(13) The separation material according to (12), wherein the separation material is for separating carbon dioxide, hydrogen, carbon monoxide, oxygen, nitrogen, hydrocarbons having from 1 to 4 carbon atoms, noble gases, hydrogen sulfide, ammonia, sulfur oxides, nitrogen oxides, siloxanes, or organic vapor.
(14) The separation material according to (12), wherein the separation material is for separating methane and carbon dioxide, hydrogen and carbon dioxide, nitrogen and carbon dioxide, ethylene and carbon dioxide, methane and ethane, ethane and ethylene, propane and propene, or air and methane.
(15) A method for separating a gas mixture using the separation material according to (12), the method comprising the step of bringing a metal complex into contact with the gas mixture in a pressure range of 0.01 to 10 MPa.
(16) The method according to (15), wherein the method is a pressure swing adsorption process or a temperature swing adsorption process.
(17) A method for producing the metal complex according to (1), comprising reacting, in a solvent, a multivalent carboxylic acid compound, at least one metal ion selected from ions of metals belonging to Groups 2 to 13 of the periodic table, an organic ligand capable of multidentate binding to the metal ion, and a monodentate organic ligand capable of binding to the metal ion to cause precipitation.

### Advantageous Effects of Invention

The present invention provides a metal complex comprising a multivalent carboxylic acid compound, at least one metal ion, an organic ligand capable of multidentate binding to the metal ion, and a monodentate organic ligand capable of binding to the metal ion.

Due to its superior adsorption performance with respect to various gases, the metal complex of the present invention can be used as an adsorbent material for adsorbing carbon dioxide, hydrogen, carbon monoxide, oxygen, nitrogen, hydrocarbons having from 1 to 4 carbon atoms, noble gases, hydrogen sulfide, ammonia, sulfur oxides, nitrogen oxides, siloxanes, organic vapor, and the like.

Further, due to its superior storage performance with respect to various gases, the metal complex of the present invention can also be used as a storage material for storing carbon dioxide, hydrogen, carbon monoxide, oxygen, nitrogen, hydrocarbons having from 1 to 4 carbon atoms, noble gases, hydrogen sulfide, ammonia, organic vapor, and the like. For example, the metal complex of the present invention can be used as a storage material for a gas storage device in a gaseous-fuel vehicle.

Furthermore, due to its superior separation performance with respect to various gases, the metal complex of the present invention can further be used as a separation material for separating carbon dioxide, hydrogen, carbon monoxide, oxygen, nitrogen, hydrocarbons having from 1 to 4 carbon atoms, noble gases, hydrogen sulfide, ammonia, sulfur oxides, nitrogen oxides, siloxanes, organic vapor, and the like. For example, the metal complex of the present invention can be used as a separation material used in a pressure swing adsorption process or temperature swing adsorption process.

### Brief Description of Drawings

Fig.1 shows a schematic diagram illustrating a jungle-gym-type framework in which 4,4'-bipyridyl is coordinated to the axial position of a metal ion in a paddle-wheel-type framework composed of a zinc ion and a carboxylate ion of terephthalic acid.
Fig.2 is a schematic diagram illustrating a three-dimensional structure in which two jungle-gym-type frameworks are interpenetrated into each other.
Fig.3 is a conceptual diagram illustrating a gaseous-fuel vehicle comprising a gas storage device.
Fig. 4 shows a powder X-ray diffraction pattern of a metal complex obtained in Synthesis Example 1.
Fig.5 is an SEM image of a metal complex obtained in Synthesis Example 1.
Fig. 6 shows a ¹H-NMR spectrum measured by dissolving the metal complex obtained in Synthesis Example 1 inammonium deuteroxide.
Fig.7 shows a powder X-ray diffraction pattern of a metal complex obtained in Synthesis Example 2.
Fig.8 is an SEM image of a metal complex obtained in Synthesis Example 2.
Fig.9 shows a powder X-ray diffraction pattern of a metal complex obtained in Synthesis Example 3.
Fig.10 is an SEM image of a metal complex obtained in Synthesis Example 3.
Fig.11 shows a powder X-ray diffraction pattern of a metal complex obtained in Comparative Synthesis Example 1.
Fig.12 is an SEM image of a metal complex obtained in Comparative Synthesis Example 1.
Fig.13 shows the water resistance evaluation results of metal complexes obtained in Synthesis Example 1 and Comparative Synthesis Example 1.
Fig.14 shows adsorption isotherms of carbon dioxide on metal complexes obtained in Synthesis Example 1 and Comparative Synthesis Example 1 at 273 K.
Fig.15 shows adsorption isotherms of carbon dioxide on metal complexes obtained in Synthesis Example 2, Synthesis Example 3, and Comparative Synthesis Example 1 at 293 K.
Fig.16 shows adsorption and desorption isotherms of methane on a metal complex obtained in Synthesis Example 1 at 298 K.
Fig.17 shows adsorption and desorption isotherms of methane on a metal complex obtained in Comparative Synthesis Example 1 at 298 K.
Fig.18 shows adsorption and desorption isotherms of carbon dioxide and methane on a metal complex obtained in Synthesis Example 1 at 293 K.

### Reference Numeral List

1. Gas storage device (fuel tank)
2. Pressure-resistant container
3. Gas storage space
4. Storage material

In the measurement results of a powder X-ray diffraction pattern, the horizontal axis represents a diffraction angle (2θ) and the vertical axis represents a diffraction intensity expressed in cps (counts per second).

In the measurement results of adsorption and desorption isotherms, the horizontal axis represents an equilibrium pressure expressed in MPa, and the vertical axis represents an equilibrium adsorption amount expressed in mL(STP)/g. In the measurement results of adsorption and desorption isotherms, the adsorption amounts (ads.) of the gases under increased pressure and the desorption amounts (des.) of the gases under decreased pressure are plotted for each pressure level. "STP" (standard temperature and pressure) denotes a state at a temperature of 273.15 K and a pressure of 1 bar (10⁵ Pa).

### Description of Embodiments

The metal complex of the present invention comprises a multivalent carboxylic acid compound, at least one metal ion selected from ions of metals belonging to Groups 2 to 13 of the periodic table, an organic ligand capable of multidentate binding to the metal ion, and a monodentate organic ligand capable of binding to the metal ion.

The multivalent carboxylic acid compound used in the present invention is not particularly limited. Dicarboxylic acid compounds, tricarboxylic acid compounds, tetracarboxylic acid compounds, etc., can be used. Examples of dicarboxylic acid compounds include succinic acid, 1,4-cyclohexanedicarboxylic acid, fumaric acid, muconic acid, 2,3-pyrazinedicarboxylic acid, isophthalic acid, terephthalic acid, 1,4-naphthalenedicarboxylic acid, 2,6-naphthalenedicarboxylic acid, 2,7-naphthalenedicarboxylic acid, 9,10-anthracenedicarboxylic acid, 4,4'-biphenyldicarboxylic acid, 4,4'-stilbenedicarboxylic acid, 2,5-pyridinedicarboxylic acid, 3,5-pyridinedicarboxylic acid, 2,5-thiophenedicarboxylic acid, 2,2'-dithiophenedicarboxylic acid, and the like. Examples of tricarboxylic acid compounds include trimesic acid, trimellitic acid, biphenyl-3,4',5-tricarboxylic acid, 1,3,5-tris(4-carboxyphenyl)benzene, 1,3,5-tris(4'-carboxy[1,1'-biphenyl]-4-yl)benzene, 2,4,6-tris(4-carboxyphenyl)-s-triazine, and the like. Examples of tetracarboxylic acid compounds include pyromellitic acid, 3,3',5,5'-tetracarboxydiphenyl methane, [1,1':4',1'']terphenyl-3,3',5,5'-tetracarboxylic acid, 1,2,4,5-tetrakis(4-carboxyphenyl)benzene, and the like. Of these, dicarboxylic acid compounds are preferable; more preferable are unsaturated dicarboxylic acid compounds (e.g., fumaric acid, 2,3-pyrazinedicarboxylic acid, isophthalic acid, terephthalic acid, 1,4-naphthalenedicarboxylic acid, 2,6-naphthalenedicarboxylic acid, 2,7-naphthalenedicarboxylic acid, 9,10-anthracenedicarboxylic acid, 4,4'-biphenyldicarboxylic acid, 4,4'-stilbenedicarboxylic acid, 2,5-pyridinedicarboxylic acid, 3,5-pyridinedicarboxylic acid, 2,5-thiophenedicarboxylic acid, and 2,2'-dithiophenedicarboxylic acid). The multivalent carboxylic acid compounds can be used singly or in a mixture of two or more. The metal complex of the present invention may be a mixture of two or more metal complexes each containing a single multivalent carboxylic acid compound (preferably dicarboxylic acid compound).

The multivalent carboxylic acid compound may further comprise a substituent other than carboxyl. The multivalent carboxylic acid having a substituent is preferably an aromatic multivalent carboxylic acid, and a substituent preferably binds to the aromatic ring of the aromatic multivalent carboxylic acid. The number of substituents is 1, 2, or 3. Examples of substituents include, but are not particularly limited to, alkyl groups (linear or branched alkyl groups having from 1 to 5 carbon atoms, such as methyl, ethyl, n-propyl, isopropyl, n-butyl, isobutyl, tert-butyl, and pentyl), halogen atoms (fluorine, chlorine, bromine, and iodine), alkoxy groups (e.g., methoxy, ethoxy, n-propoxy, isopropoxy, n-butoxy, isobutoxy, and tert-butoxy), amino groups, monoalkylamino groups (e.g., methylamino), dialkylamino groups (e.g., dimethylamino), formyl groups, epoxy groups, acyloxy groups (e.g., acetoxy, n-propanoyloxy, n-butanoyloxy, pivaloyloxy, and benzoyloxy), alkoxycarbonyl groups (e.g., methoxycarbonyl, ethoxycarbonyl, and n-butoxycarbonyl), nitro groups, cyano groups, hydroxyl groups, acetyl groups, trifluoromethyl groups, and the like. Specific examples include multivalent carboxylic acid compounds having a substituent such as 2-nitroterephthalic acid, 2-fluoroterephthalic acid, 1,2,3,4-tetrafluoro terephthalic acid, 2,4,6-trifluoro-1,3,5-benzenetricarboxylic acid, and the like.

The multivalent carboxylic acid compound may be used in the form of acid anhydride or alkali metal salt.

In the present invention, at least one metal ion selected from ions of metals belonging to Groups 2 to 13 of the periodic table is used. The ions of metals belonging to Group 2 of the periodic table include beryllium, magnesium, calcium, strontium, barium, and radium ions. The ions of metals belonging to Group 3 of the periodic table include scandium, yttrium, lanthanide, and actinoid ions. The ions of metals belonging to Group 4 of the periodic table include titanium, zirconium, hafnium, and rutherfordium ions. The ions of metals belonging to Group 5 of the periodic table include vanadium, niobium, tantalum, and dubnium ions. The ions of metals belonging to Group 6 of the periodic table include chromium, molybdenum, tungsten, and seaborgium ions. The ions of metals belonging to Group 7 of the periodic table include manganese, technetium, rhenium, and bohrium ions. The ions of metals belonging to Group 8 of the periodic table include iron, ruthenium, osmium, and hassium ions. The ions of metals belonging to Group 9 of the periodic table include cobalt, rhodium, iridium, and meitnerium ions. The ions of metals belonging to Group 10 of the periodic table include nickel, palladium, platinum, and darmstadtium ions. The ions of metals belonging to Group 11 of the periodic table include copper, silver, gold, and roentgenium ions. The ions of metals belonging to Group 12 of the periodic table include zinc, cadmium, mercury, and copernicium ions. The ions of metals belonging to Group 13 of the periodic table include boron, aluminum, gallium, indium, thallium, and ununtrium ions.

Examples of ions of metals belonging to Groups 2 to 13 of the periodic table preferably used in the present invention include magnesium, calcium, scandium, lanthanide (e.g., lantern, terbium, and lutetium), actinoid (e.g., actinium and lawrencium), zirconium, vanadium, chromium, molybdenum, manganese, iron, cobalt, nickel, copper, zinc, cadmium, aluminum, and like ions. Of these, manganese, cobalt, nickel, copper, and zinc ions are preferable, and copper ions are particularly preferable. It is preferable to use a single metal ion; however, it is also possible to use two or more metal ions. The metal complex of the present invention may be a mixture of two or more metal complexes each containing a single metal ion.

The metal ion can be used in the form of metal salt. Usable examples of metal salts include magnesium salts, calcium salts, scandium salts, lanthanide salts (e.g., lantern salt, terbium salt, and lutetium salt), actinoid salts (e.g., actinium salt, and lawrencium salt), zirconium salts, vanadium salts, chromium salts, molybdenum salts, manganese salts, iron salts, cobalt salts, nickel salts, copper salts, zinc salts, cadmium salts, and aluminum salts. Of these, manganese salts, cobalt salts, nickel salts, copper salts, and zinc salts are preferable, and copper salts are more preferable. It is preferable to use a single metal salt; however, it is also possible to mix two or more metal salts. Examples of such metal salts include organic acid salts such as acetates and formates, and inorganic acid salts such as sulfates, nitrates, hydrochlorides, hydrobromates, and carbonates.

The organic ligand capable of multidentate binding used in the present invention refers to a neutral ligand having at least two sites coordinated to a metal ion with a lone electron pair.

Examples of sites coordinated to a metal ion with a lone electron pair include a nitrogen atom, an oxygen atom, a phosphorus atom, a sulfur atom, and the like. The organic ligand capable of multidentate binding is preferably a heterocyclic compound, in particular, a heterocyclic compound that has nitrogen atoms as coordination sites. The heterocyclic compound may have a substituent, or may be bound to a divalent hydrocarbon group (for example, a divalent group in which two hydrogen atoms are removed from ethyne).

The organic ligand capable of multidentate binding to the meal ion used in the present invention is not particularly limited; however, organic ligands capable of bidentate binding to the metal ion, organic ligands capable of tridentate binding to the metal ion, organic ligands capable of tetradentate binding to the metal ion, etc., can be used. Examples of organic ligands capable of bidentate binding (bidentate organic ligands) include 1,4-diazabicyclo[2.2.2]octane, pyrazine, 2,5-dimethylpyrazine, 4,4'-bipyridyl, 2,2'-dimethyl-4,4'-bipyridine, 1,2-bis(4-pyridyl)ethyne, 1,4-bis(4-pyridyl)butadiyne, 1,4-bis(4-pyridyl)benzene, 3,6-di(4-pyridyl)-1,2,4,5-tetrazine, 2,2'-bi-1,6-naphthyridine, phenazine, diazapyrene, 2,6-di(4-pyridyl)-benzo[1,2-c:4,5-c']dipyrrole-1,3,5,7(2H,6H)-tetrone, N,N'-di(4-pyridyl)-1,4,5,8-naphthalene tetracarboxy diimide, trans-1,2-bis(4-pyridyl)ethene, 4,4'-azopyridine, 1,2-bis(4-pyridyl)ethane, 4,4'-dipyridyl sulfide, 1,3-bis(4-pyridyl)propane, 1,2-bis(4-pyridyl)-glycol, N-(4-pyridyl)isonicotinamide, 1,2-bis(1-imidazolyl)ethane, 1,2-bis(1,2,4-triazolyl)ethane, 1,2-bis (1,2,3,4-tetrazolyl)ethane, 1,3-bis(1-imidazolyl)propane, 1,3-bis(1,2,4-trizolyl)propane, 1,3-bis(1,2,3,4-tetrazolyl)propane, 1,4-bis(4-pyridyl)butane, 1,4-bis(1-imidazolyl)butane, 1,4-bis(1,2,4-triazolyl)butane, 1,4-bis(1,2,3,4-tetrazolyl)butane, 1,4-bis(benzimidazole-1-ylmethyl)-2,4,5,6-tetramethyl benzene, 1,4-bis(4-pyridylmethyl)-2,3,5,6-tetramethyl benzene, 1,3-bis(imidazole-1-ylmethyl)-2,4,6-trimethyl benzene, 1,3-bis(4-pyridylmethyl)-2,4,6-trimethyl benzene, and the like. Examples of organic ligands capable of tridentate binding (tridentate organic ligands) include 1,3,5-tris(2-pyridyl)benzene, 1,3,5-tris(3-pyridyl)benzene, 1,3,5-tris(4-pyridyl)benzene, 1,3,5-tris(1-imidazolyl)benzene, 2,4,6-tris(2-pyridyl)-1,3,5-triazine, 2,4,6-tris(3-pyridyl)-1,3,5-triazine, 2,4,6-tris(4-pyridyl)-1,3,5-triazine, 2,4,6-tris(1-imidazolyl)-1,3,5-triazine, and the like. Examples of organic ligands capable of tetradentate binding (tetradentate organic ligands) include 1,2,4,5-tetrakis(2-pyridyl)benzene, 1,2,4,5-tetrakis(3-pyridyl)benzene, 1,2,4,5-tetrakis(4-pyridyl)benzene, 1,2,4,5-tetrakis(1-imidazolyl)benzene, tetrakis(3-pyridyloxymethylene)methane, tetrakis(4-pyridyloxymethylene) methane, tetrakis(1-imidazolylmethyl)methane, and the like. The organic ligands capable of multidentate binding can be used singly or in a mixture of two or more. The metal complex of the present invention may be a mixture of two or more metal complexes each containing a single organic ligand capable of multidentate binding.

Of the organic ligands capable of multidentate binding, organic ligands capable of bidentate binding are preferable. For example, more preferably used are 1,4-diazabicyclo[2.2.2]octane, pyrazine, 2,5-dimethylpyrazine, 4,4'-bipyridyl, 2,2'-dimethyl-4,4'-bipyridine, 1,2-bis(4-pyridyl)ethyne, 1,4-bis(4-pyridyl)butadiyne, 1,4-bis(4-pyridyl)benzene, 3,6-di(4-pyridyl)-1,2,4,5-tetrazine, 2,2'-bi-1,6-naphthyridine, phenazine, diazapyrene, 2,6-di(4-pyridyl)-benzo[1,2-c:4,5-c']dipyrrole-1,3,5,7(2H,6H)-tetrone, N,N'-di(4-pyridyl)-1,4,5,8-naphthalene tetracarboxy diimide, trans-1,2-bis(4-pyridyl)ethene, 4,4'-azopyridine, 1,2-bis(4-pyridyl)ethane, 4,4'-dipyridyl sulfide, 1,3-bis(4-pyridyl)propane, 1,2-bis(4-pyridyl)-glycol, N-(4-pyridyl)isonicotinamide, and the like.

The organic ligand capable of multidentate binding used in the present invention is preferably an organic ligand capable of bidentate binding that belongs to the D_{∞h} point group and has a longitudinal length of 7.0 Å or more and 16.0 Å or less.

The point group to which the organic ligand capable of bidentate binding belongs may be determined according to the method disclosed in Reference Document 1 below. Reference Document 1: Bunshino Taisho to Gunron (Molecular Symmetry and Group Theory; Masao Nakazaki, 1973, Tokyo Kagaku Dojin Co., Ltd.) pp.39-40.

For example, since 4,4'-bipyridyl, 1,2-bis(4-pyridyl)ethyne, 2,7-diazapyrene, 1,4-bis(4-pyridyl)benzene, 3,6-di(4-pyridyl)-1,2,4,5-tetrazine, 2,6-di(4-pyridyl)-benzo[1,2-c:4,5-c']dipyrrole-1,3,5,7(2H,6H)-tetorone, 4,4'-bis(4-pyridyl)biphenylene, N,N'-di(4-pyridyl)-1,4,5,8-naphthalene tetracarboxy diimide, and the like are bilaterally symmetric linear molecules having a symmetric center, they belong to the D_{∞h} point group. Further, since 1,2-bis(4-pyridyl)ethene has a twofold axis and symmetric planes perpendicular to the axis, it belongs to the C₂ₕ point group.

When the point group of the organic ligand capable of bidentate binding is D_{∞h}, the high symmetry reduces wasteful gaps. Thus, high adsorption performance can be exhibited. In addition, when the longitudinal length of the organic ligand capable of bidendate binding is 7.0 Å or more and 16.0 Å or less, the distance between the metal ions in the metal complex will be suitable. Thus, a metal complex having optimal gaps for adsorbing and desorbing a gas molecule can be formed. The metal complex can be obtained even when the longitudinal length of the organic ligand capable of bidentate binding falls out of the above range; however, adsorption performance, storage performance, and separation performance tend to decrease.

The longitudinal length of the organic ligand capable of bidentate binding in the present specification is defined as the distance between two atoms having the longest distance between them, among the atoms coordinated to the metal ion in the structural formula, in the most stable structure found by structure optimization according to the PM5 semiempirical molecular orbital method after the conformational analysis according to the MM3 molecular dynamics method using Scigress Explorer Professional Version 7.6.0.52 (produced by Fujitsu).

For example, the interatomic distance between nitrogen atoms of 1,4-diazabicyclo[2.2.2]octane is 2.609 Å, the interatomic distance between nitrogen atoms of pyrazine is 2.810 Å, the interatomic distance between nitrogen atoms of 4,4'-bipyridyl is 7.061 Å, the interatomic distance between nitrogen atoms of 1,2-bis(4-pyridyl)ethyne is 9.583 Å, the interatomic distance between nitrogen atoms of 1,4-bis(4-pyridyl)benzene is 11.315 Å, the interatomic distance between nitrogen atoms of 3,6-di(4-pyridyl)-1,2,4,5-tetrazine is 11.204 Å, the interatomic distance between nitrogen atoms of 2,6-di(4-pyridyl)-benzo[1,2-c:4,5-c']dipyrrole-1,3,5,7(2H,6H)-tetrone is 15.309 Å, the interatomic distance between nitrogen atoms of 4,4'-bis(4-pyridyl)biphenyl is 15.570 Å, and the interatomic distance between nitrogen atoms of N,N'-di(4-pyridyl)-1,4,5,8-naphthalene tetracarboxy diimide is 15.533 Å. Of the organic ligands capable of bidentate binding that belong to the D_{∞h} point group, and have a longitudinal length of 7.0 Å or more and 16.0 Å or less, 4,4'-bipyridyl is particularly preferably used.

The monodentate organic ligand used in the present invention refers to a neutral ligand having one site coordinated to a metal ion with a lone electron pair. Examples of monodentate organic ligands include substituted or unsubstituted furan, thiophene, pyridine, quinoline, isoquinoline, acridine, triphenyl phosphine, triphenyl phosphite, methylisocyanide, and the like. Of these, a monodentate organic ligand comprising a pyridine ring as part of the chemical structure, and pyridine is particularly preferable.

The monodentate organic ligand preferably has a C₁₋₂₃ hydrocarbon group as a substituent. Examples of hydrocarbon groups include linear or branched alkyl groups such as methyl, ethyl, n-propyl, isopropyl, n-butyl, isobutyl, and tert-butyl; and aryl groups such as phenyl and naphthyl. In the hydrocarbon group, one or two or more hydrogen atoms may be substituted with a fluorine atom, chlorine atom, bromine atom, or iodine atom.

Usable examples of monodentate organic ligands having a C₁₋₂₃ hydrocarbon group as a substituent include substituted pyridines such as methyl pyridines, tert-butyl pyridines, phenyl pyridines, and trifluoromethyl pyridines; substituted quinolines such as methyl quinolines and tert-butyl quinolines; substituted isoquinolines such as methyl isoquinolines; triphenylphosphines; and the like. Of these, substituted pyridines are preferable, and 4-methylpyridine, 4-tert-butylpyridine, and 4-phenylpyridine are more preferably used. The monodentate organic ligands can be used singly or as a mixture of two or more. The metal complex of the present invention may be a mixture of two or more metal complexes each containing a single monodentate organic ligand. The monodentate organic ligand may be present from the early stage of the reaction, or may be added in the latter stage of the reaction (for example, after reacting components other than the monodentate organic ligand).

The composition ratio of the organic ligand capable of multidentate binding to the monodentate organic ligand incorporated in the metal complex of the present invention is preferably such that organic ligand capable of multidentate binding : monodentate organic ligand = 1:10 to 5,000:1, more preferably 1:20 to 5,000:1, even more preferably 20:1 to 5,000:1, and particularly preferably 100:1 to 2,500:1. When the composition ratio of the multidentate organic ligand and monodentate organic ligand is in this range, the metal complex can exhibit higher water resistance and higher adsorption and desorption performance.

The composition ratio of the organic ligand capable of multidentate binding and the monodentate organic ligand composing the metal complex of the present invention can be determined by analysis using, for example, gas chromatography, high-performance liquid chromatography, or NMR after the metal complex is dissolved to obtain a homogeneous solution. However, the method is not limited to these.

The metal complex of the present invention may further include a monocarboxylic acid compound other than the above constituents. The monocarboxylic acid compound can further improve the water resistance of the metal complex. Usable examples of monocarboxylic acid compounds include formic acid; aliphatic monocarboxylic acids such as acetic acid, propionic acid, butyric acid, isobutyric acid, valeric acid, caproic acid, enanthic acid, cyclohexane carboxylic acid, caprylic acid, octylic acid, pelargonic acid, capric acid, lauric acid, myristic acid, pentadecyl acid, palmitic acid, margaric acid, stearic acid, tuberculostearic acid, arachidic acid, behenic acid, lignoceric acid, α-linoleic acid, eicosapentaenoic acid, docosahexaenoic acid, linolic acid, and oleic acid; aromatic monocarboxylic acids such as benzoic acid; complex aromatic monocarboxylic acids such as nicotinic acid and isonicotinic acid; and the like. Among these, formic acid, acetic acid, octylic acid, lauric acid, myristic acid, palmitic acid, and stearic acid are preferable.

The monocarboxylic acid compound may be used in the form of acid anhydride or alkali metal salt. The monocarboxylic acid compound can be incorporated in the metal complex structure of the present invention when used as a counteranion of the raw material metal salt. For example, when a copper ion and acetic acid are respectively used as a metal ion and a monocarboxylic acid compound, copper acetate can be used as the raw material metal salt. The monocarboxylic acid compound may be present from the early stage of the reaction, or may be added in the latter stage of the reaction.

When the metal complex of the present invention includes the monocarboxylic acid compound, the proportion of the monocarboxylic acid compound is not particularly limited as long as the effect of the present invention is not impaired. For example, the composition ratio of the multivalent carboxylic acid compound to the monocarboxylic acid compound is preferably such that multivalent carboxylic acid compound: monocarboxylic acid compound = 10:1 to 5,000:1, more preferably 50:1 to 5,000:1, even more preferably 100:1 to 5,000:1, and particularly preferably 100:1 to 2,500:1.

The composition ratio of the multivalent carboxylic acid compound to the monocarboxylic acid compound composing the metal complex of the present invention can be determined by analysis using, for example, gas chromatography, high-performance liquid chromatography, or NMR after the metal complex is dissolved to form a homogeneous solution. However, the method is not limited to these.

The metal complex of the present invention can be produced by reacting a multivalent carboxylic acid compound, at least one metal salt selected from salts of metals belonging to Groups 2 to 13 of the periodic table, an organic ligand capable of multidentate binding to the metal ion, a monodentate organic ligand capable of binding to the metal ion, and optionally a monocarboxylic acid compound in vapor phase, liquid phase, or solid phase. The metal complex of the present invention is preferably produced by reacting these components in a solvent under ordinary pressure for several hours to several days, and precipitating them. The reaction may be performed under ultrasonic or microwave irradiation. For example, the metal complex of the present invention can be obtained by mixing and reacting a metal salt aqueous solution or an organic solvent solution with an aqueous solution or an organic solvent solution containing a multivalent carboxylic acid compound, an organic ligand capable of multidentate binding, and a monodentate organic ligand capable of binding to the metal ion, under ordinary pressure.

The mixing ratio of the metal salt to the multivalent carboxylic acid compound during the manufacture of the metal complex is preferably in the following molar ratio: metal salt : multivalent carboxylic acid compound = 1:5 to 8:1, and more preferably 1:3 to 6:1. If the mixing ratio falls out of this range upon the reaction, the yield decreases and side reaction increases, even though the target metal complex can be obtained.

The mixing ratio of the metal salt to the organic ligand capable of multidentate binding during the manufacture of the metal complex is preferably in the following molar ratio: metal salt : organic ligand capable of multidentate binding = 1:3 to 3:1, and more preferably 1:2 to 2:1. If the mixing ratio falls out of this range, the yield of the target metal complex decreases, and residues of unreacted raw materials are generated, thereby causing complication in the purification process of the resulting metal complex.

The molar concentration of the multivalent carboxylic acid compound in the mixed solution used for the manufacture of the metal complex is preferably 0.01 to 5.0 mol/L, and more preferably 0.05 to 2.0 mol/L. If the molar concentration falls below this range upon the reaction, the yield of reaction undesirably decreases even though the target metal complex can still be obtained. If the molar concentration falls above this range upon the reaction, the solubility decreases, thereby hindering the smooth progress of reaction.

The molar concentration of the metal salt in the mixed solution used for the manufacture of the metal complex is preferably 0.01 to 5.0 mol/L, and more preferably 0.05 to 2.0 mol/L. If the molar concentration falls below this range upon the reaction, the yield of reaction undesirably decreases even though the target metal complex can still be obtained. If the molar concentration falls above this range, residues of unreacted metal salts are generated, thereby causing complication in the purification process of the resulting metal complex.

The molar concentration of the organic ligand capable of multidentate binding in the mixed solution used for the manufacture of the metal complex is preferably 0.005 to 2.5 mol/L, and more preferably 0.025 to 1.0 mol/L. If the molar concentration falls below this range upon the reaction, the yield of reaction undesirably decreases even though the target metal complex can still be obtained. If the molar concentration falls above this range upon the reaction, the solubility decreases, thereby hindering the smooth progress of reaction.

The solvent used for the manufacture of metal complex may be an organic solvent, water, or a mixed solvent of these. Specific examples of solvents include methanol, ethanol, propanol, diethylether, dimethoxyethane, tetrahydrofuran, hexane, cyclohexane, heptane, benzene, toluene, methylene chloride, chloroform, acetone, ethyl acetate, acetonitrile, N,N-dimethylformamide, water, and mixed solvents of these substances. The reaction temperature is preferably 253 to 423 K, and more preferably 298 to 423 K.

The completion of the reaction may be confirmed by analyzing the remaining amount of the raw materials by using absorption spectrophotometry, gas chromatography, or high-performance liquid chromatography; however, the method is not limited to these. After the reaction is completed, the resulting mixture is subjected to suction filtration to collect the precipitates. The precipitates are washed with an organic solvent and dried in vacuum for several hours at about 373 K, thereby obtaining the metal complex of the present invention.

Since the metal complex of the present invention is a porous material and can adsorb and desorb a low molecular compound such as gas in the micropores, it can be used as an adsorbent material, storage material, and separation material for various gases. However, the metal complex does not adsorb gas when a solvent used in manufacture is adsorbed. Accordingly, when the metal complex is used as the adsorbent material, storage material, or separation material of the present invention, it is necessary to dry the metal complex under vacuum in advance to remove the solvent in the micropores. The vacuum drying may be generally performed at a temperature that does not decompose the metal complex (e.g., 293 K to 523 K or less); however, an even lower temperature (e.g., 293 K to 393 K or less) is preferable. This operation may be replaced by washing with supercritical carbon dioxide, which is more efficient.

The metal complex of the present invention has a one-dimensional, two-dimensional, or three-dimensional framework, depending on the type of multivalent carboxylic acid compound, metal ion, and organic ligand capable of multidentate binding to the metal ion to be used. The framework of the metal complex can be confirmed by using single-crystal X-ray structure analysis, powder X-ray crystal structure analysis, or single-crystal neutron structure analysis; however, the method is not limited to these.

Examples of frameworks of the metal complex include a three-dimensional structure in which two jungle-gym-type frameworks are interpenetrated into each other; a three-dimensional structure composed of two-dimensional-sheet-type frameworks obtained by using a copper ion as a metal ion, 2,5-dihydroxybenzoate ion as a multivalent carboxylic acid compound, and 4,4'-bipyridyl as an organic ligand capable of multidentate binding; and the like.

The particle size or morphology of the metal complex of the present invention can be controlled according to the type and amount of the monodentate organic ligand used.

The particle size of the metal complex can be confirmed by using a laser diffraction method, a dynamic light scattering method, an imaging method, a settling method, or the like; however, the method is not limited to these.

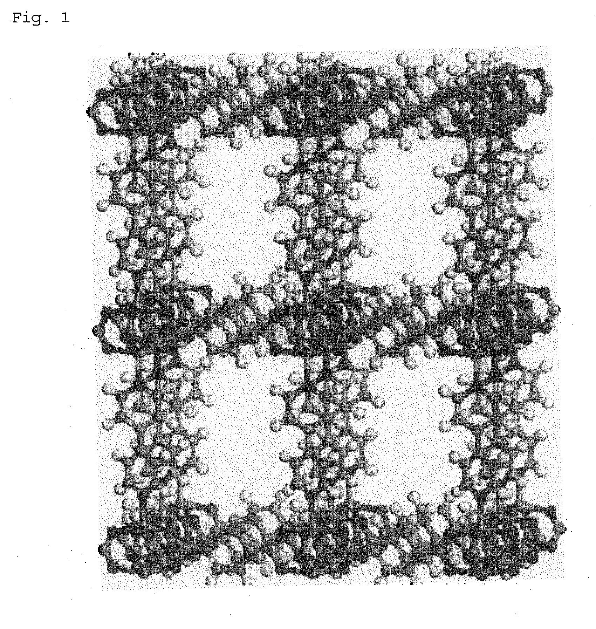

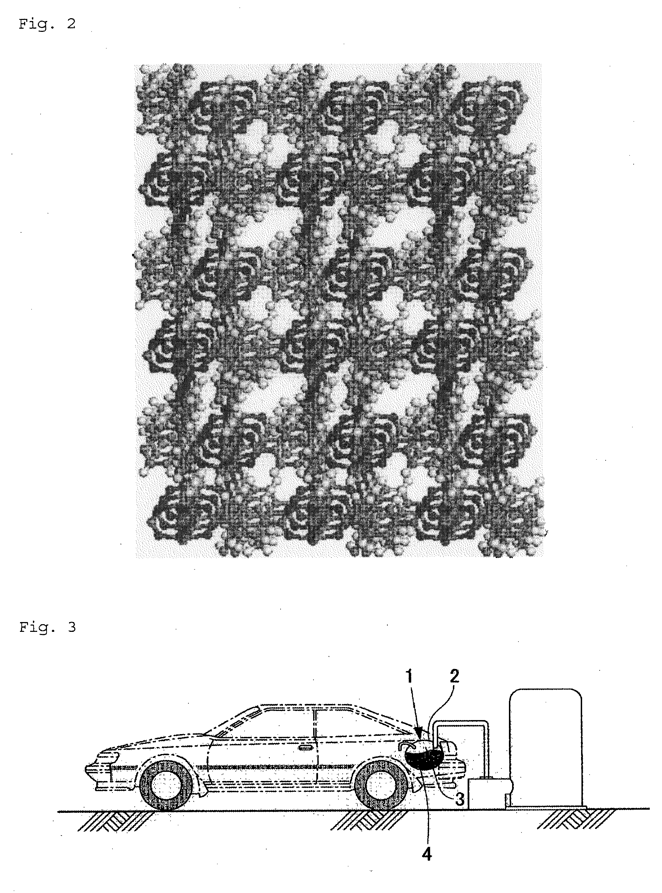

One detailed example is a metal complex comprising terephthalic acid as a multivalent carboxylic acid compound, zinc ion as a metal ion, and 4,4'-bipyridyl as an organic ligand capable of multidentate binding. The metal complex of the present invention thus obtained has a three-dimensional structure composed of interpenetrated two jungle-gym-type frameworks. The jungle-gym-type framework is structured such that 4,4'-bipyridyl is coordinated to the axial position of a metal ion in a paddle-wheel-type framework composed of a metal ion and a carboxylate ion of terephthalic acid. Fig. 1 is a schematic diagram illustrating a jungle-gym-type framework, and Fig. 2 is a schematic diagram illustrating a three-dimensional structure in which two jungle-gym-type frameworks are interpenetrated into each other.

The "jungle-gym-type framework" is defined as a jungle-gym-like three-dimensional structure in which an organic ligand capable of multidentate binding is coordinated to the axial position of a metal ion in a paddle-wheel-type framework composed of a metal ion and a multivalent carboxylic acid compound such as terephthalic acid, thus connecting the two-dimensional lattice sheets composed of the multivalent carboxylic acid compound and the metal ion. "A structure in which multiple jungle-gym-type frameworks are interpenetrated into each other" is defined as a three-dimensional framework in which multiple jungle-gym-type frameworks are interpenetrated into each other by filling each other's micropores.

For example, single-crystal X-ray structure analysis, powder X-ray crystal structure analysis, and single-crystal neutron structure analysis may be used to confirm whether the metal complex has the structure in which multiple jungle-gym-type frameworks are interpenetrated into each other; however, the method is not limited to these.

By reacting the organic ligand capable of multidentate binding with the metal ion in the presence of the monodentate organic ligand, the reaction of the metal ion and the organic ligand capable of multidentate binding, and the reaction of the metal ion and the monodentate organic ligand will compete with each other. The monodentate organic ligand can be considered to be a terminator for crystal growth reaction; however, since the reaction of the metal ion and the monodentate organic ligand is reversible, the crystal nucleation rate and the crystal growth rate are controlled. Consequently, the particle size or morphology can be adjusted.

When coordinated, since the monodentate organic ligand has only one coordination site, the crystal growth at the coordination site stops, thus adjusting the particle size and morphology. Taking crystal growth mechanism into consideration, the monodentate organic ligand is disproportionately present at the crystal growth end, i.e., at the crystal surface. The monodentate organic ligand disproportionately present at the crystal surface of the metal complex can be confirmed, for example, by time-of-flight secondary ion mass spectrometry.

When the monodentate organic ligand has a C₁₋₂₃ hydrocarbon group as a substituent, a hydrocarbon chain derived from the monodentate organic ligand is exposed to the crystal surface of the metal complex, and the hydrophobic effect and the steric effect of the hydrocarbon group prevent water from approaching the metal ion. Accordingly, the metal complex has further improved water resistance.

The above water resistance improvement mechanism is estimated. Even if water resistance improvement mechanism does not conform to the above mechanism, it will be covered within the technical scope of the present invention insofar as it satisfies the requirements specified in the present invention.

The water resistance of the metal complex in the present invention can be evaluated, for example, by measuring the change in adsorption amount before and after exposure to water vapor.

The metal complex of the present invention has excellent adsorption performance, storage performance, and separation performance with respect to various gases. Accordingly, the metal complex of the present invention is useful as an adsorbent material, a storage material, or a separation material for various gases, which are also within the technical scope of the present invention.

The metal complex of the present invention is useful as an adsorbent material, a storage material, or a separation material for adsorbing, storing, or separating carbon dioxide, hydrogen, carbon monoxide, oxygen, nitrogen, hydrocarbons having from 1 to 4 carbon atoms (such as methane, ethane, ethylene, acetylene, propane, propene, methylacetylene, propadiene, butadiene, 1-butene, isobutene, 1-butyne, 2-butyne, 1,3-butadiene, and methylallene), noble gases (such as helium, neon, argon, krypton, and xenon), hydrogen sulfide, ammonia, sulfur oxides, nitrogen oxides, siloxanes (such as hexamethylcyclotrisiloxane and octamethylcyclotetrasiloxane), water vapor, and organic vapor. The separation material of the present invention is suitable for separating methane and carbon dioxide, hydrogen and carbon dioxide, nitrogen and carbon dioxide, ethylene and carbon dioxide, methane and ethane, ethylene and ethane, nitrogen and oxygen, oxygen and argon, nitrogen and methan, or air and methane by using a pressure swing adsorption process or a temperature swing adsorption process.

The term "organic vapor" means a vaporizing gas of an organic substance that is in liquid form at ordinary temperature under ordinary pressure. Examples of such organic substances include alcohols, such as methanol and ethanol; amines, such as trimethylamine; aldehydes, such as formaldehyde and acetaldehyde; aliphatic hydrocarbons having 5 to 16 carbon atoms, such as pentane, isoprene, hexane, cyclohexane, heptane, methylcyclohexane, octane, 1-octene, cyclooctane, cyclooctene, 1,5-cyclooctadiene, 4-vinyl-1-cyclohexene, and 1,5,9-cyclododecatriene; aromatic hydrocarbons, such as benzene and toluene; ketones, such as acetone and methyl ethyl ketone; esters, such as methyl acetate and ethyl acetate; and halogenated hydrocarbons, such as methyl chloride and chloroform.

The adsorbent material, storage material, and separation material of the present invention can be molded optionally using a binder such as titanium dioxide, silica dioxide, aluminum oxide, montmorillonite, kaolin, bentonite, halloysite, dickite, nacrite, anauxite, tetraalkoxysilane (e.g., tetramethoxysilane, tetraethoxysilane, tetrapropoxysilane, and tetrabutoxysilane), starch, cellulose, cellulose acetate, polyvinyl alcohol, polyamide, polyester, polycarbonate, polysulfone, polyethersulfone, polyolefin, polytetrafluoroethylene, elastomer, etc., within a range that does not impair the effect of the present invention. The adsorbent material, storage material, and separation material may also be combined with natural or synthetic fibers such as paper, or inorganic fibers such as glass or alumina.

Of these, an elastomer is preferably used as a binder in view of molding properties. The elastomer used is not particularly limited, and any elastomer can be used. In particular, thermoplastic elastomers are preferable. Usable thermoplastic elastomers are block copolymers having, as part of the polymer chain, at least one polymer block (rubber phase) with a glass transition temperature of 273 K or less. Particularly preferable thermoplastic elastomers are block copolymers having, as part of the polymer chain, a polymer block (rubber phase) with a glass transition temperature of 273 K or less, and a polymer block (constrained phase) with a glass transition temperature of 310 K or more.

Examples of thermoplastic elastomers include styrene elastomers, olefin elastomers, urethane elastomers, polyester elastomers, nitrile elastomers, amide elastomers, polybutadiene elastomers, acrylic elastomers, and vinyl chloride elastomers. Of these, styrene elastomers, olefin elastomers, and acrylic elastomers are particularly preferably used. The mixing ratio of the metal complex to the elastomer is not particularly limited; however, it is preferably in the following mass ratio: metal complex : elastomer = 1:99 to 99:1, more preferably 10:90 to 90:10.

The adsorbent material, storage material, and separation material of the present invention can be used in any form. The metal complex can be used in the form of powders without any treatment, or can be formed into pellets, films, sheets, plates, pipes, tubes, rods, granules, various special molded products, fibers, hollow filaments, woven fabrics, knitted fabrics, non-woven fabrics, and the like.

The method for producing pellets comprising the metal complex, or the adsorbent material, storage material, or separation material, of the present invention is not particularly limited, and any known pelletizing method can be used. To produce pellets with a higher density, tablet compression is preferable. In tablet compression, the metal complex, or the composition of the metal complex and a binder, is generally prepared in advance, and then solidified in a specific shape under pressure using a commercially available tablet compression machine. During this process, a lubricant such as black lead and magnesium stearate may be added to the preparation product, as necessary.

The method for producing sheets comprising the adsorbent material, storage material, or separation material of the present invention is not particularly limited, and any known sheet-forming method can be used. To produce sheets with a higher density, wet paper making is preferable. Wet paper making is a method in which raw materials are dispersed in water and filtered through a net, followed by drying.

An example of special molded products is a honeycomb shape. Any known processing method can be used to form sheets comprising the adsorbent material, storage material, or separation material of the present invention into a honeycomb shape. "Honeycomb shape" as mentioned in the present invention refers to a shape of continuous hollow polygonal columns with a hexagonal cross section, as well as a square, sine-wave, or roll cross section; or to a shape of continuous hollow cylindrical columns, such as cylinders. For example, sheets comprising the adsorbent material, storage material, or separation material of the present invention are formed into a sine-wave honeycomb shape in the following manner. First, a sheet comprising the adsorbent material of the present invention is passed through shaping rolls to form a wave-shaped sheet, and a flat sheet is bonded to one or both sides of the wave-shaped sheet. These sheets are laminated to form a sine-wave honeycomb filter. It is common to secure the sheets with an adhesive that is put on the top of the wave shapes. However, when wave-shaped sheets comprising the adsorbent material of the present invention are laminated, a flat sheet placed between the wave-shaped sheets is necessarily secured, and so an adhesive is not necessarily used. When an adhesive is used, one that does not impair the adsorption performance of the sheets must be used. Usable adhesives are, for example, corn starch, vinyl acetate resin, and acrylic resin. The gas adsorption performance of the wave-shaped sheet comprising the composition of the present invention can be enhanced by reducing the adhesion pitch of the sheet and lowering the thread height of the sheet. The pitch is preferably 0.5 to 8 mm, and the thread height is preferably 0.4 to 5 mm.

By taking advantage of its storage performance, the metal complex of the present invention (or the adsorbent material of the present invention) can be used in gas storage devices. One example of the gas storage devices is a gas storage device that comprises a pressure-resistant container that can be hermetically sealed and has an inlet and outlet for gas, wherein the pressure-resistant container has a gas storage space, and wherein the adsorbent material comprising the metal complex of the present invention is placed in the gas storage space. A desired gas is stored in the gas storage device by compressing the gas into the gas storage device so that the gas is adsorbed by the storage material placed in the device. The gas is taken out from the gas storage device by opening a pressure valve to reduce the internal pressure in the pressure-resistant container, thereby desorbing the gas. When the storage material is placed in the gas storage space, powders of the metal complex of the present invention can be used. In terms of handling properties, etc., pellets obtained by molding the metal complex of the present invention may be used.

The gas storage device 1, as described above, can store fuel gas in a gas storage space 3, and can be suitably used as a fuel tank 1 of, for example, a gaseous-fuel vehicle. Fig. 3 shows an example of a gaseous-fuel vehicle comprising the gas storage device of the present invention. The gaseous-fuel vehicle comprises as a fuel tank 1, the above gas storage device 1, in which the metal complex of the present invention is placed, and also comprises an engine as an internal combustion engine that receives natural gas stored in the fuel tank 1 and mixes the natural gas with oxygen-containing gas for combustion (e.g., air), thereby obtaining running drive force by combustion of the gas mixture. The fuel tank 1 comprises a pressure-resistant container 2, an inlet/outlet pair for enabling a gas to be stored to enter or exit, and a pair of valves each provided in the outlet and inlet and constituting a hermetically sealed mechanism that can maintain the gas in the container 2 in a pressurized state. The fuel tank 1 is filled with fuel (natural gas) in a pressurized state at a gas station. The fuel tank 1 is internally provided with a storage material 4 comprising the metal complex of the present invention. The storage material 4 adsorbs the natural gas (e.g., gas comprising methane as a main component) at ordinary temperature under increased pressure. When the valve on the outlet side is opened, the adsorbed gas is desorbed from the storage material 4 and transmitted to the engine side such that the gas is combusted to generate running drive force.

The fuel tank 1, which is internally provided with the storage material comprising the metal complex of the present invention, has higher gas compressibility relative to the apparent pressure, compared to a fuel tank without the storage material. The thickness of the tank can be thereby reduced, and the weight of the entire gas storage device can also be reduced, which is advantageous, for example, for gaseous fuel vehicles. The fuel tank 1 is generally maintained at ordinary temperature without cooling. When the temperature increases, e.g., during summer, the temperature of the tank becomes relatively high. The storage material of the present invention is able to maintain its high storage ability in such a high temperature range (about 298 to 333 K) and is therefore useful.

The separation method comprises a step of bringing a gas and the metal complex (or the separation material) of the present invention into contact with each other under a condition that enables the gas to be adsorbed to the metal complex. The condition, i.e., the adsorption pressure and the adsorption temperature that enable the gas to be adsorbed to the metal complex can be suitably set according to the type of the material to be adsorbed. For example, the adsorption pressure is preferably 0.01 to 10 MPa, and more preferably 0.1 to 3.5 MPa. The adsorption temperature is preferably 195 to 343 K, and more preferably 273 to 313 K.

The pressure swing adsorption process or the temperature swing adsorption process may be used as the separation method. When performing the pressure swing adsorption process as the separation method, the separation method further comprises a step of increasing the pressure from an adsorption pressure to a pressure enabling the gas to be desorbed from the metal complex. The desorption pressure may be suitably set according to the type of the material to be adsorbed. For example, the desorption pressure is preferably 0.005 to 2 MPa, and more preferably 0.01 to 0.1 MPa. When performing the temperature swing adsorption process as the separation method, the separation method further comprises a step of increasing the temperature from an adsorption temperature to a temperature enabling the gas to be desorbed from the metal complex. The desorption temperature can be suitably set according to the type of the material to be adsorbed. For example, desorption temperature is preferably 303 to 473 K, and more preferably 313 to 373 K.

When performing the pressure swing adsorption process or the temperature swing adsorption process as the separation method, the step of bringing the gas to be in contact with the metal complex and the step of changing the pressure or the temperature that enables the gas to be desorbed from the metal complex may be appropriately repeated.

### Examples

The invention will hereinafter be described specifically by using examples. It should be borne in mind, however, that the invention is not limited to or limited by these examples. The analysis and evaluation in the following Examples and Comparative Examples were conducted as described below.

### (1) Powder X-Ray Diffraction Pattern Measurement

The powder X-ray diffraction pattern was measured using an X-ray diffractometer based on the symmetric reflection method while scanning at a scanning rate of 1°/min within a diffraction angle (2θ) range of from 5 to 50°. Details of the analysis conditions are shown below.

### ▪ Analysis Conditions

Apparatus: Smart Lab produced by Rigaku Corporation
X-ray source: Cu Kα (λ=1.5418 A) 45 kV 200 mA
Goniometer: Vertical Goniometer
Detector: D/teX Ultra
Step width: 0.02°
Slit: Divergence slit = 2/3°
Receiving slit = 0.3 mm
Scattering slit = 2/3°

### (2) Quantification of Monodentate Organic Ligand

The metal complex was dissolved in ammonium deuteroxide to prepare a homogeneous solution, and ¹H NMR measurement was performed. The monodentate organic ligand in the metal complex was quantified from the integrated ratio of the resulting spectrum. Details of the analysis conditions are shown below.

### ▪ Analysis Conditions

Apparatus: Advance 600 produced by Bruker Biospin K.K.
Resonance frequency: 600 MHz
Solvent: ammonium deuteroxide
Standard substance: Sodium 3-(Trimethylsilyl)propionate-2,2,3,3-d₄
Temperature: 298 K
Pulse repetition time: 5.5 seconds
Scans: 2,000 times

### (3) Observation Using SEM

The metal complex obtained after conduction treatment was observed using a scanning electron microscope. Details of the analysis conditions are shown below.

### ▪ Analysis Conditions

Apparatus: S-3000N produced by Hitachi High Technologies Corporation
Accelerating voltage: 10.0 kV

### (4) Measurement of Adsorption Isotherm or Adsorption and Desorption Isotherms

The amounts of gas adsorbed and desorbed were measured according to the volumetric method by using a high-pressure gas adsorption measuring instrument to plot adsorption and desorption isotherms (in accordance with JIS Z8831-2). Before the measurement, the sample was dried at 373 K and 0.5 Pa for 5 hours to remove adsorbed water and the like. The following are details of the analysis conditions.

### ▪ Analysis Conditions

Apparatus: BELSORP-HP produced by Bel Japan, Inc.
Equilibrium waiting time: 500 s

### Synthesis Example 1

Under nitrogen atmosphere, 5.86 g (23.5 mmol) of copper sulfate pentahydrate, 3.90 g (23.5 mmol) of terephthalic acid, and 32.4 g (704 mmol) of formic acid were dissolved in 3,750 mL of methanol. The mixture was stirred at 313 K for 24 hours. After collecting the precipitated metal complex by suction filtration, the metal complex was washed three times with methanol. Subsequently, the collected metal complex was dispersed in methanol (2,000 mL) under nitrogen atmosphere, and 1.83 g (11.7 mmol) of 4,4'-bipyridyl and 31.7 g (235 mmol) of 4-tert-butylpyridine were added thereto. The mixture was stirred at 298 K for 3 hours, during which the reaction solution remained suspended. After collecting the metal complex by suction filtration, the metal complex was washed three times with methanol. Subsequently, the mixture was dried at 373 K and 50 Pa for 8 hours, thereby obtaining 1.70 g of the target metal complex.

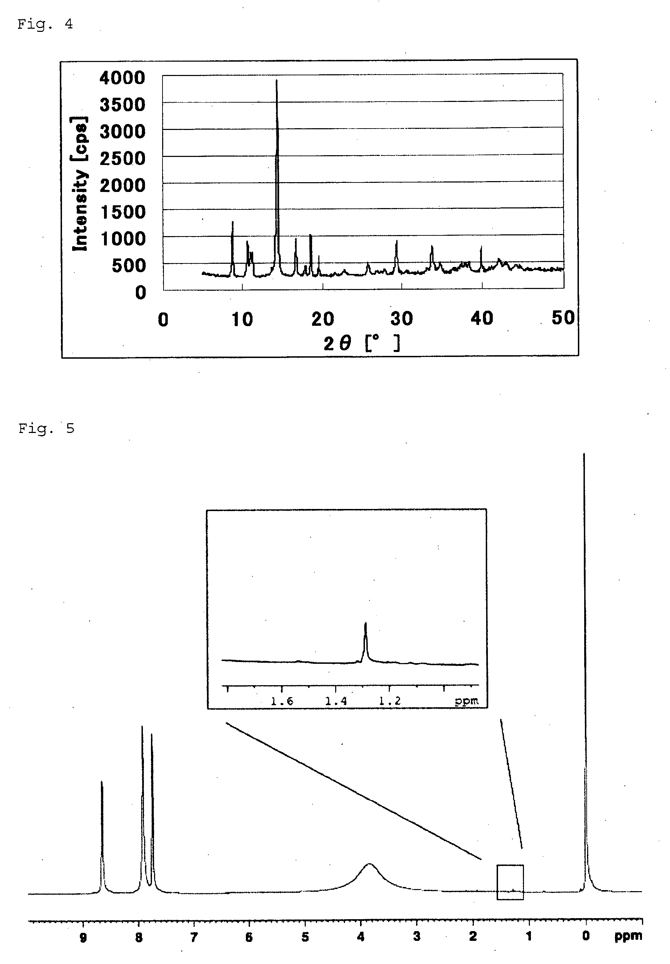

The powder X-ray diffraction pattern of the resulting metal complex is shown in Fig. 4. Comparison from the simulation pattern obtained based on the structure analysis results of the separately synthesized single crystal reveals that the resulting metal complex has a structure in which two jungle-gym-type frameworks are interpenetrated into each other. The single crystal structure analysis results are shown below.
Triclinic (P-1)
a=7.898(3)Å
b=8.930(3)Å
c=10.818(3)Å
α=67.509(12)°
β=80.401(14)°
γ=79.566(13)°
V=689.3(4)Å³
R=0.0330
Rw=0.0905

10 mg of the resulting metal complex was dissolved in 700 mg of heavy ammonia water (containing 0.4 wt% Sodium 3-(Trimethylsilyl)propionate-2,2,3,3-d₄ as a standard substance) to perform ¹H NMR measurement. The resulting spectrum is shown in Fig. 5. As a result of spectrum analysis, the peak integral value attributed to protons of the tert-butyl group of the 4-tert-butylpyridine at 1.288 ppm (s, 9H) was 8.919 when the peak integral value attributed to protons at positions 2, 6, 2' and 6' of the 4,4'-bipyridil at 8.659 ppm (s, 4H) was taken as 1,000. This indicates that the molar ratio of the 4,4'-bipyridyl and 4-tert-butylpyridine contained in the metal complex is such that 4,4'-bipyridyl:4-tert-butylpyridine = 252:1. In Fig. 5, the broad signal around at 3.9 ppm is attributed to water.

The results of the single crystal X-ray structure analysis and ¹H NMR measurement reveal that the composition formula of the resulting metal complex is [Cu₂(C₈H₄O₄)₂(C₁₀H₈N₂)₁₋ₓ(C₄H₉C₅H₄N)ₓ]ₙ (x = 0.0040). n is a positive integer. Since the value x was small, the theoretical yield was calculated based on the molecular weight of the compound represented by [Cu₂(C₈H₄O₄)₂(C₁₀H₈N₂)]ₙ (copper: terephthalic acid : 4,4'-bipyridyl =2:2:1). Consequently, the yield of the resulting metal complex was 24%.

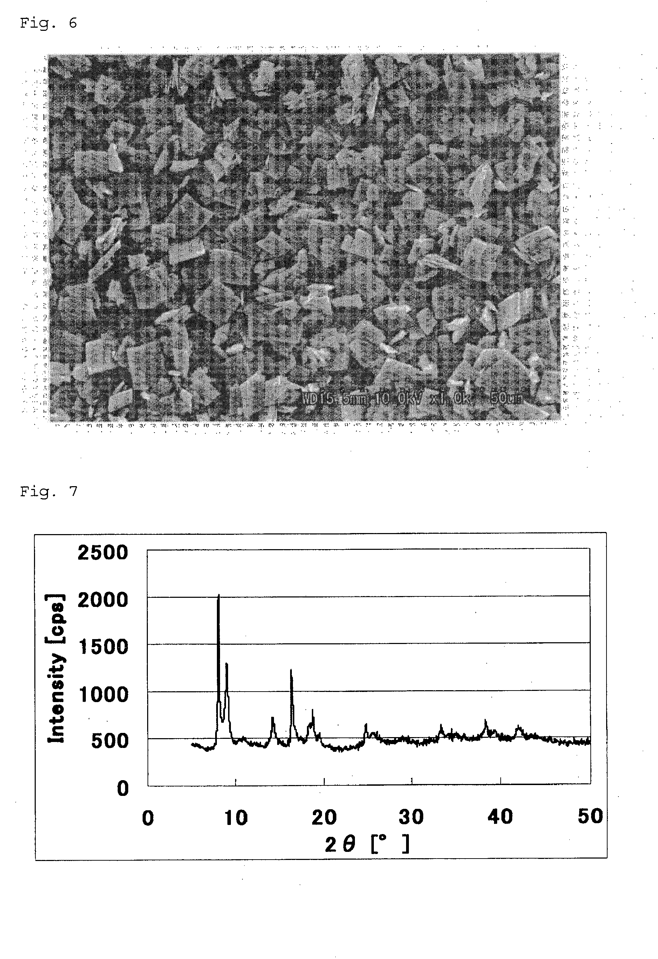

The SEM image of the resulting metal complex is shown in Fig. 6 (magnification: 1000x).

### Synthesis Example 2

Under nitrogen atmosphere, 5.86 g (23.5 mmol) of copper sulfate pentahydrate, 3.90 g (23.5 mmol) of terephthalic acid, and 32.4 g (704 mmol) of formic acid were dissolved in 3,750 mL of methanol. The mixture was stirred at 313 K for 24 hours. After collecting the precipitated metal complex by suction filtration, the metal complex was washed three times with methanol. Subsequently, the collected metal complex was dispersed in methanol (2,000 mL) under nitrogen atmosphere, and 1.83 g (11.7 mmol) of 4,4'-bipyridyl and 21.8 g (235 mmol) of 4-methyl pyridine were added thereto. The mixture was stirred at 298 K for 3 hours, during which the reaction solution remained suspended. After collecting the metal complex by suction filtration, the metal complex was washed three times with methanol. Subsequently, the mixture was dried at 373 K and 50 Pa for 8 hours, thereby obtaining 1.22 g of the target metal complex.

The powder X-ray diffraction pattern of the resulting metal complex is shown in Fig. 7.

¹H NMR measurement was conducted in the same manner as in Synthesis Example 1. As a result of spectrum analysis, the peak integral value attributed to protons at positions 2 and 5 of the 4-methylpyridine at 8.474 ppm (s, 2H) was 7.497 when the peak integral value attributed to protons at positions 2, 6, 2' and 6' of the 4,4'-bipyridil at 8.659 ppm (s, 4H) was taken as 1,000. This indicates that the molar ratio of the 4,4'-bipyridyl and 4-methylpyridine contained in the metal complex is such that 4,4'-bipyridyl:4-methylpyridine = 1:15.

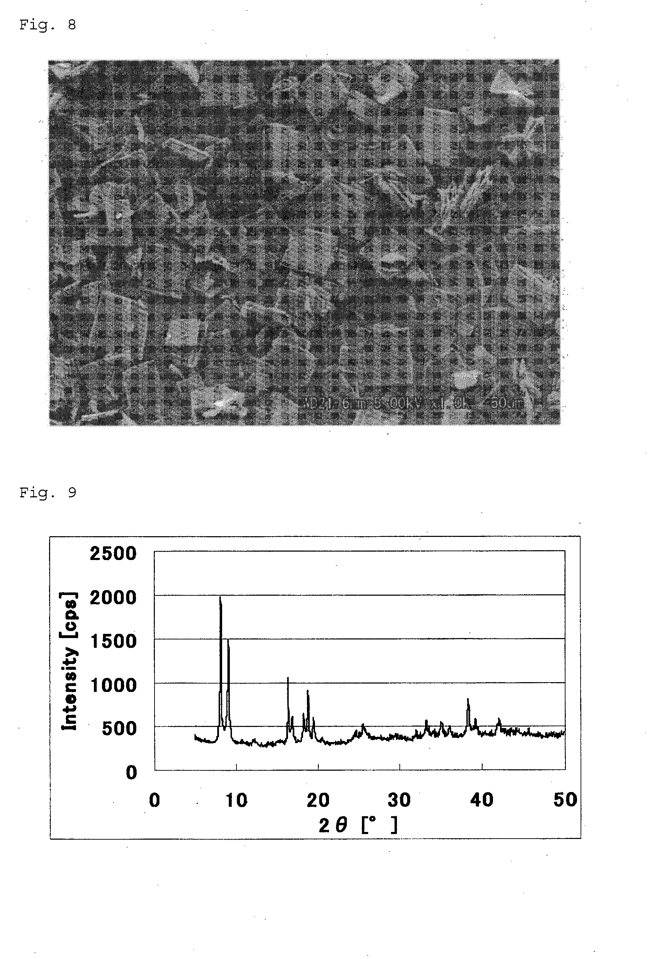

The SEM image of the resulting metal complex is shown in Fig. 8 (magnification: 1000x).

### Synthesis Example 3

Under nitrogen atmosphere, 5.86 g (23.5 mmol) of copper sulfate pentahydrate, 3.90 g (23.5 mmol) of terephthalic acid, and 32.4 g (704 mmol) of formic acid were dissolved in 3,750 mL of methanol. The mixture was stirred at 313 K for 24 hours. After collecting the precipitated metal complex by suction filtration, the metal complex was washed three times with methanol. Subsequently, the collected metal complex was dispersed in methanol (2,000 mL) under nitrogen atmosphere, and 1.83 g (11.7 mmol) of 4,4'-bipyridyl and 30.3 g (235 mmol) of isoquinoline were added thereto. The mixture was stirred at 298 K for 3 hours, during which the reaction solution remained suspended. After collecting the metal complex by suction filtration, the metal complex was washed three times with methanol. Subsequently, the mixture was dried at 373 K and 50 Pa for 8 hours, thereby obtaining 1.04 g of the target metal complex.

The powder X-ray diffraction pattern of the resulting metal complex is shown in Fig. 9.

¹H NMR measurement was conducted in the same manner as in Synthesis Example 1. As a result of spectrum analysis, the peak integral value attributed to a proton at position 3 of the isoquinoline at 8.495 ppm (s, 1H) was 404 when the peak integral value attributed to protons at positions 2, 6, 2' and 6' of the 4,4'-bipyridil at 8.659 ppm (s, 4H) was taken as 1,000. This indicates that the molar ratio of the 4,4'-bipyridyl and isoquinoline contained in the metal complex is such that 4,4'-bipyridyl:isoquinoline = 1:1.6.

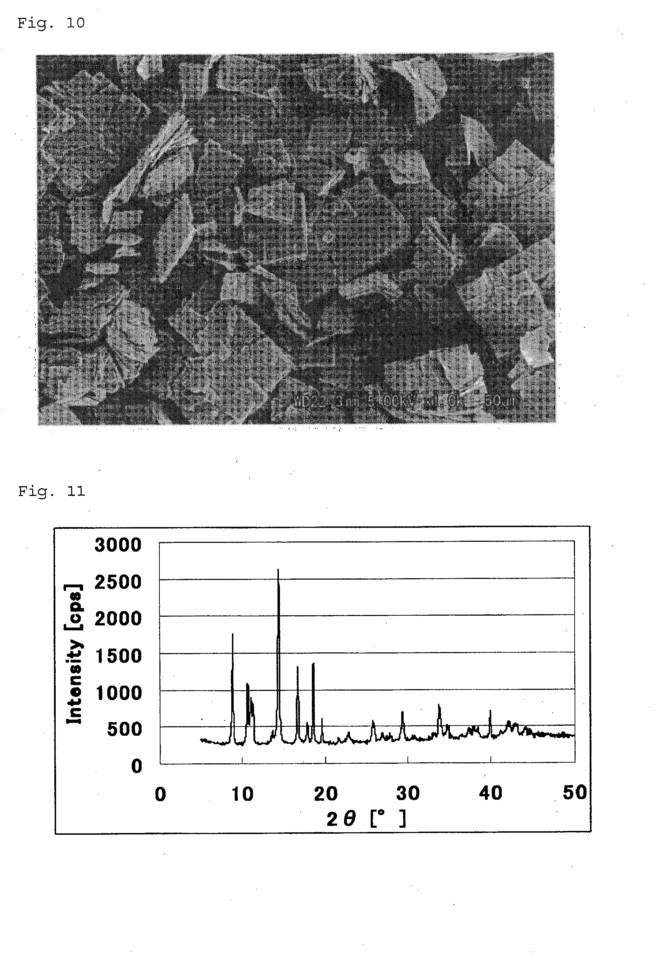

The SEM image of the resulting metal complex is shown in Fig. 10.

### Comparative Synthesis Example 1

Under nitrogen atmosphere, 5.86 g (23.5 mmol) of copper sulfate pentahydrate, 3.90 g (23.5 mmol) of terephthalic acid, and 32.4 g (704 mmol) of formic acid were dissolved in 3,750 mL of methanol. The mixture was stirred at 313 K for 24 hours. After collecting the precipitated metal complex by suction filtration, the metal complex was washed three times with methanol. Subsequently, the collected metal complex was dispersed in methanol (2,000 mL) under nitrogen atmosphere, and 1.83 g (11.7 mmol) of 4,4'-bipyridyl was added thereto. The mixture was stirred at 298 K for 3 hours, during which the reaction solution remained suspended. After collecting the metal complex by suction filtration, the metal complex was washed three times with methanol. Subsequently, the mixture was dried at 373 K and 50 Pa for 8 hours, thereby obtaining 1.79 g (yield: 25%) of the target metal complex.

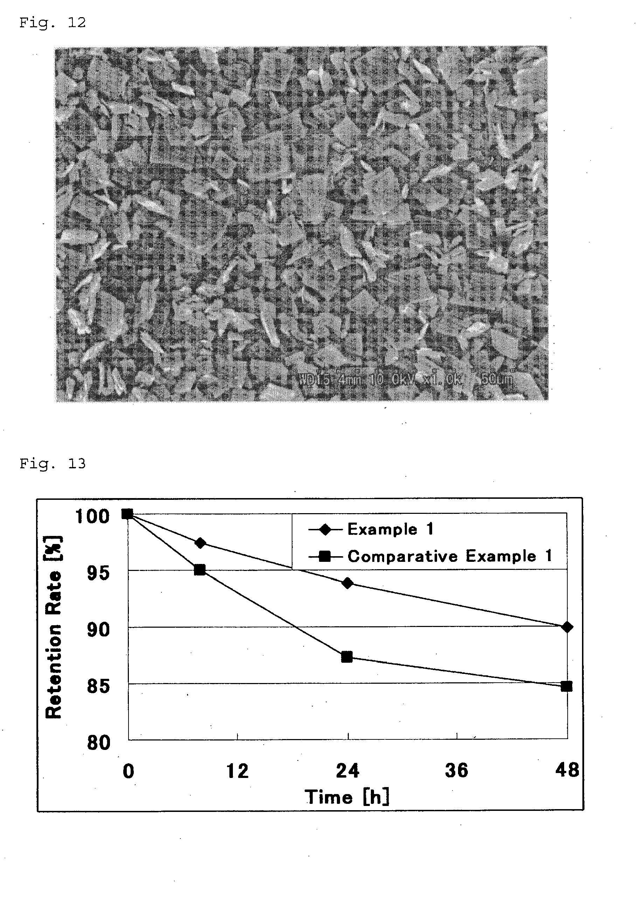

The powder X-ray diffraction pattern of the resulting metal complex is shown in Fig. 11. Comparison from the simulation pattern obtained based on the structure analysis results of the separately synthesized single crystal reveals that the resulting metal complex has a structure in which two jungle-gym-type frameworks, which are the same as those of the metal complex obtained in Synthesis Example 1, are interpenetrated into each other. The SEM image of the resulting metal complex is shown in Fig. 12.

### Example 1

Using the low-temperature constant temperature and humidity PL-2KP produced by Espec Corporation, the metal complex obtained in Synthesis Example 1 was placed under an atmosphere of 353 K and a relative humidity of 80% to perform a water vapor exposure test. The sampling was performed after 8 hours, 24 hours, and 48 hours, and the amount of carbon dioxide adsorbed at 273 K was measured according to the volumetric method to plot an adsorption isotherm. The equilibrium adsorption amount of carbon dioxide at 0.92 MPa was calculated from the adsorption isotherm, and the change in retention rate was plotted. The results are shown in Fig. 13.

### Comparative Example 1

Using the low-temperature constant temperature and humidity PL-2KP produced by Espec Corporation, the metal complex obtained in Comparative Synthesis Example 1 was placed under an atmosphere of 353 K and a relative humidity of 80% to perform a vapor exposure test. The sampling was performed after 8 hours, 24 hours, and 48 hours, and the amount of carbon dioxide adsorbed at 273 K was measured according to the volumetric method to plot an adsorption isotherm. The equilibrium adsorption amount of carbon dioxide at 0.92 MPa was calculated from the adsorption isotherm, and the change in retention rate was plotted. The results are shown in Fig. 13.

Fig. 13 reveals that the metal complex obtained in Synthesis Example 1, which satisfies the constituent features of the present invention, has a higher carbon dioxide equilibrium adsorption retention rate even under high temperature and high humidity, and less decrease in the retention rate over time than the metal complex obtained in Comparative Synthesis Example 1, which does not satisfy the constituent features of the present invention. This clearly indicates that the metal complex of the present invention has excellent water resistance.

### Example 2

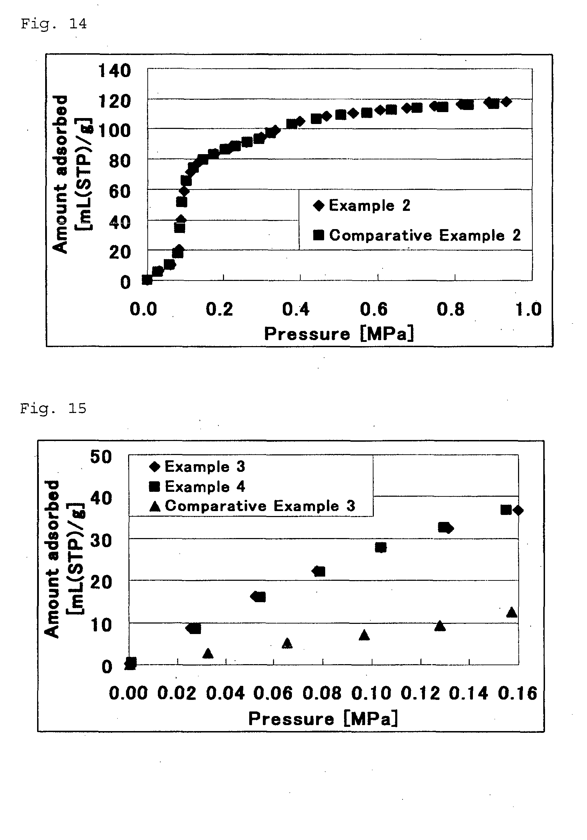

The amount of carbon dioxide adsorbed at 273 K by the metal complex obtained in Synthesis Example 1 was measured according to the volumetric method to plot adsorption isotherm. The results are shown in Fig. 14.

### Comparative Example 2

The amount of carbon dioxide adsorbed at 273 K by the metal complex obtained in Comparative Synthesis Example 1 was measured according to the volumetric method to plot adsorption isotherm. The results are shown in Fig. 14.

Fig. 14 reveals that the carbon dioxide adsorption performance of the metal complex obtained in Synthesis Example 1 is equal to that of the metal complex obtained in Comparative Synthesis Example 1.

The results of Examples 1 and 2 and Comparative Examples 1 and 2 reveal that the metal complex obtained in Synthesis Example 1, which satisfies the constituent features of the present invention and has a monodentate organic ligand, maintains the same level of carbon dioxide adsorption performance and has improved water resistance, compared to the metal complex obtained in Comparative Synthesis Example 1, which does not have a monodentate organic ligand. The reason for such a difference is not certain; however, excellent water resistance was exhibited presumably because 4-tert-butylpyridine composing the metal complex of the present invention was disproportionately present on the surface of crystal, and the tert-butyl group of the 4-tert-butylpyridine provides hydrophobic properties, consequently inhibiting vapor diffusion into micropores.

### Example 3

The amount of carbon dioxide adsorbed at 293 K by the metal complex obtained in Synthesis Example 2 was measured according to the volumetric method to plot an adsorption isotherm. The results are shown in Fig. 15.

### Example 4

The amount of carbon dioxide adsorbed at 293 K by the metal complex obtained in Synthesis Example 3 was measured according to the volumetric method to plot an adsorption isotherm. The results are shown in Fig. 15.

### Comparative Example 3

The amount of carbon dioxide adsorbed at 293 K by the metal complex obtained in Comparative Synthesis Example 1 was measured according to the volumetric method to plot an adsorption isotherm. The results are shown in Fig. 15.

Fig. 15 reveals that since the metal complexes obtained in Synthesis Examples 2 and 3, which satisfy the constituent features of the present invention, have a larger carbon dioxide adsorption amount than the metal complex obtained in Comparative Synthesis Example 1, which does not satisfy the constituent features of the present invention, the metal complexes obtained in Synthesis Examples 2 and 3 are excellent as an adsorbent material for carbon dioxide.

### Example 5

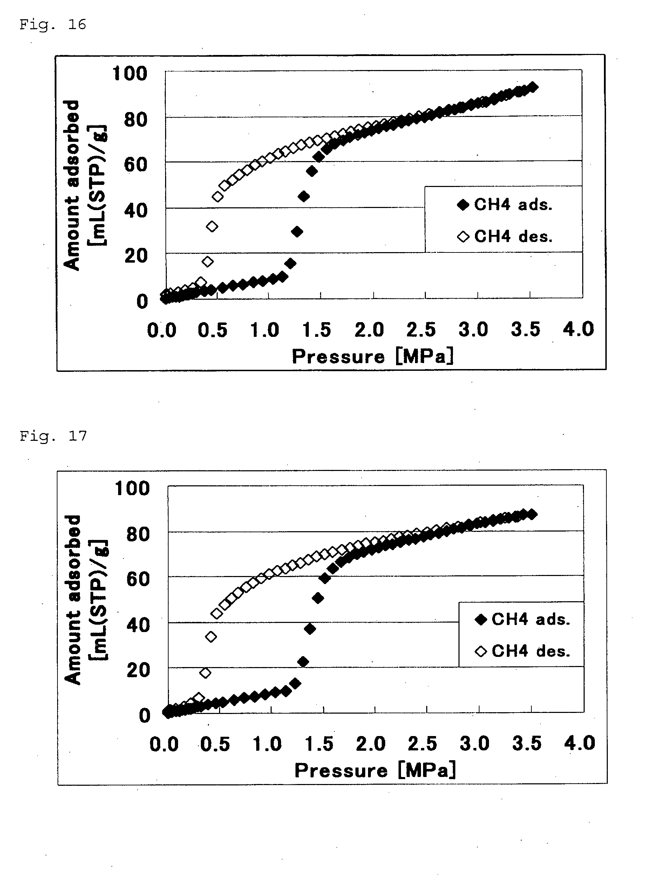

The amounts of methane adsorbed and desorbed at 298 K by the metal complex obtained in Synthesis Example 1 were measured according to the volumetric method to plot adsorption and desorption isotherms. The results are shown in Fig. 16.

### Comparative Example 4

The amounts of methane adsorbed and desorbed at 298 K by the metal complex obtained in Comparative Synthesis Example 1 were measured according to the volumetric method to plot adsorption and desorption isotherms. The results are shown in Fig. 17.

Comparison between Figs. 16 and 17 reveals that the methane adsorption performance of the metal complex obtained in Synthesis Example 1 is equal to that of the metal complex obtained in Comparative Synthesis Example 1.

The results of Examples 1 and 5 and Comparative Examples 1 and 4 reveal that the metal complex obtained in Synthesis Example 1, which satisfies the constituent features of the present invention, maintains the same level of methane adsorption performance and has improved water resistance, compared to the metal complex obtained in Comparative Synthesis Example 1, which does not have a monodentate organic ligand.

Fig. 16 reveals that the metal complex obtained in Synthesis Example 1, which satisfies the constituent features of the present invention, has a high effective methane storage amount because it adsorbs methane along with the increase in pressure, and desorbs 95% or more of the methane adsorbed along with the decrease in pressure without decreasing the pressure to 0.1 MPa or less; thus, it is expected to be applied for fuel storage tanks of gaseous-fuel vehicles.

### Example 6

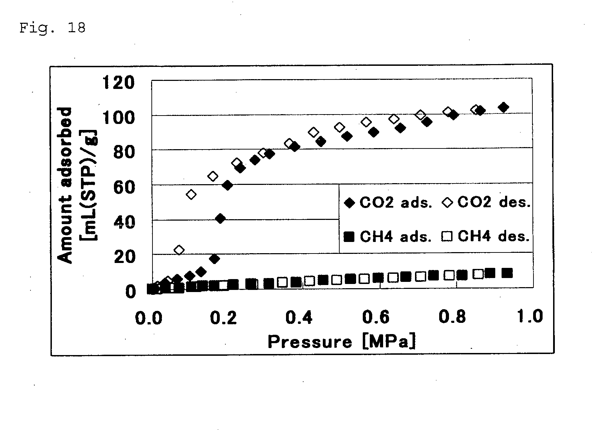

The amounts of carbon dioxide and methane adsorbed and desorbed at 293 K by the metal complex obtained in Synthesis Example 1 were measured according to the volumetric method to plot adsorption and desorption isotherms. The results are shown in Fig. 18.

Comparison between Figs. 16 and 18 reveals that the metal complex obtained in Synthesis Example 1, which satisfies the constituent features of the present invention, has excellent performance of separating methane and carbon dioxide because the adsorption starting pressures of methane and carbon dioxide greatly varies. By taking advantage of its properties, the metal complex of the present invention can be preferably used for separation according to the pressure swing adsorption process.

## Claims

1. A metal complex comprising:
a multivalent carboxylic acid compound,
at least one metal ion selected from ions of metals belonging to Groups 2 to 13 of the periodic table,
an organic ligand capable of multidentate binding to the metal ion, and
a monodentate organic ligand capable of binding to the metal ion.

2. The metal complex according to claim 1, wherein the monodentate organic ligand has a C₁₋₂₃ hydrocarbon group as a substituent.

3. The metal complex according to claim 2, wherein the monodentate organic ligand is a pyridine having a C₁₋₂₃ hydrocarbon group as a substituent.

4. The metal complex according to any one of claims 1 to 3, wherein the multivalent carboxylic acid compound is a dicarboxylic acid compound.

5. The metal complex according to any one of claims 1 to 4, wherein the organic ligand capable of multidentate binding is at least one member selected from 1,4-diazabicyclo[2.2.2]octane, pyrazine, 2,5-dimethylpyrazine, 4,4'-bipyridyl, 2,2'-dimethyl-4,4'-bipyridine, 1,2-bis(4-pyridyl)ethyne, 1,4-bis(4-pyridyl) butadiyne, 1,4-bis(4-pyridyl)benzene, 3,6-di(4-pyridyl)-1,2,4,5-tetrazine, 2,2'-bi-1,6-naphthyridine, phenazine, diazapyrene, 2,6-di(4-pyridyl)-benzo[1,2-c:4,5-c']dipyrrole-1,3,5,7(2H,6H)-tetrone, N,N'-di(4-pyridyl)-1,4,5,8-naphthalene tetracarboxlic diimide, trans-1,2-bis(4-pyridyl)ethene, 4,4'-azopyridine, 1,2-bis(4-pyridyl)ethane, 4,4'-dipyridyl sulfide, 1,3-bis(4-pyridyl)propane, 1,2-bis(4-pyridyl)-glycol, and N-(4-pyridyl)isonicotinamide.

6. An adsorbent material comprising the metal complex according to any one of claims 1 to 5.

7. The adsorbent material according to claim 6, wherein the adsorbent material is for adsorbing carbon dioxide, hydrogen, carbon monoxide, oxygen, nitrogen, hydrocarbons having from 1 to 4 carbon atoms, noble gases, hydrogen sulfide, ammonia, sulfur oxides, nitrogen oxides, siloxanes, or organic vapor.

8. A storage material comprising the metal complex according to any one of claims 1 to 5.

9. The storage material according to claim 8, wherein the storage material is for storing carbon dioxide, hydrogen, carbon monoxide, oxygen, nitrogen, hydrocarbons having from 1 to 4 carbon atoms, noble gases, hydrogen sulfide, ammonia, or organic vapor.

10. A gas storage device comprising a pressure-resistant container that can be hermetically sealed and that has an inlet and outlet for gas, the pressure-resistant container having a gas storage space therein, and the storage material according to claim 8 being placed in the gas storage space.

11. A gaseous-fuel vehicle comprising an internal combustion engine that obtains driving force from fuel gas supplied from the gas storage device according to claim 10.

12. A separation material comprising the metal complex according to any one of claims 1 to 5.

13. The separation material according to claim 12, wherein the separation material is for separating carbon dioxide, hydrogen, carbon monoxide, oxygen, nitrogen, hydrocarbons having from 1 to 4 carbon atoms, noble gases, hydrogen sulfide, ammonia, sulfur oxides, nitrogen oxides, siloxanes, or organic vapor.

14. The separation material according to claim 12, wherein the separation material is for separating methane and carbon dioxide, hydrogen and carbon dioxide, nitrogen and carbon dioxide, ethylene and carbon dioxide, methane and ethane, ethane and ethylene, propane and propene, or air and methane.

15. A method for separating a gas mixture using the separation material according to claim 12, the method comprising the step of bringing a metal complex into contact with the gas mixture in a pressure range of 0.01 to 10 MPa.

16. The method according to claim 15, wherein the method is a pressure swing adsorption process or a temperature swing adsorption process.

17. A method for producing the metal complex according to claim 1, comprising reacting, in a solvent, a multivalent carboxylic acid compound, at least one metal ion selected from ions of metals belonging to Groups 2 to 13 of the periodic table, an organic ligand capable of multidentate binding to the metal ion, and a monodentate organic ligand capable of binding to the metal ion to cause precipitation.
